# EUROPÄISCHE PATENTANMELDUNG

(11) **EP 4 664 078 A1**
(43) Veröffentlichungstag der Anmeldung: **17.12.2025**
(21) Anmeldenummer: 24181825.1
(22) Anmeldetag: 12.06.2024
(51) Int. Cl.: G01F 22/00, A61M 1/00, A61M 5/00, G01N 29/00

(54) **MEDIZINISCHE VORRICHTUNG**

(71) Anmelder: B. Braun Melsungen AG, 34212 Melsungen (DE)
(72) Erfinder: KAUBA, Michael, 34277 Fuldabrück (DE); MORITZEN, Hans-Christian, 34119 Kassel (DE); ROSENKRANZ, Heiko, 34593 Knüllwald (DE)
(74) Vertreter: Winter, Brandl - Partnerschaft mbB

(57) **Zusammenfassung**

Offenbart ist eine medizinische Vorrichtung (1), die aufweist: eine Oszillationseinheit (2), die mit einem Abschnitt (3) einer Flüssigkeitsleitung (4) mittelbar oder unmittelbar in Kontakt bringbar ist, eine Ansteuereinheit (9), die ausgebildet ist, durch Anlegen einer elektrischen Spannung, insbesondere einer Wechselspannung, die Oszillationseinheit (2) zu einer Schwingung anzuregen, und eine Auswerteinheit (10), die ausgebildet ist, eine Impedanz der angeregten Oszillationseinheit (2) zu bestimmen und ein Gasvolumen (6) in dem Abschnitt (3) der Flüssigkeitsleitung (4) unter Berücksichtigung der Impedanz zu bestimmen.

## Beschreibung

### Technisches Gebiet

Die vorliegende Offenbarung betrifft eine medizinische Vorrichtung, die es ermöglicht ein Gasvolumen in einem Abschnitt einer Flüssigkeitsleitung zu bestimmen.

Es ist Aufgabe der vorliegenden Offenbarung, eine medizinische Vorrichtung bereitzustellen, die ein Gasvolumen in einem Abschnitt einer Flüssigkeitsleitung bestimmen kann. Insbesondere soll nicht nur bestimmt werden, ob Gas in der Flüssigkeitsleitung vorhanden ist, sondern es soll auch eine Bestimmung eines Volumens des Gases in der Flüssigkeitsleitung ermöglicht werden.

Diese Aufgabe wird durch die medizinische Vorrichtung gemäß Anspruch 1 gelöst. Vorteilhafte Weiterbildungen der vorliegenden Offenbarung sind Gegenstand von Unteransprüchen und/oder sind in der nachfolgenden Beschreibung und/oder den Figuren offenbart.

Eine offenbarungsgemäße medizinische Vorrichtung weist eine Oszillationseinheit, eine Ansteuereinheit und eine Auswerteeinheit auf. Die Oszillationseinheit ist mit einem Abschnitt einer Flüssigkeitsleitung mittelbar oder unmittelbar in Kontakt bringbar. Die Ansteuereinheit ist ausgebildet, durch Anlegen einer elektrischen Spannung, insbesondere einer Wechselspannung, die Oszillationseinheit zu einer Schwingung anzuregen. Die Auswerteeinheit ist ausgebildet, eine Impedanz der angeregten Oszillationseinheit zu bestimmen und ein Gasvolumen in dem Abschnitt der Flüssigkeitsleitung unter Berücksichtigung der Impedanz zu bestimmen.

Demnach wird das Gasvolumen in dem Abschnitt der Flüssigkeitsleitung anhand der Impedanz der elektrisch angeregten Oszillationseinheit bestimmt, deren elektromechanisches Verhalten vom Gasvolumen in dem Abschnitt der Flüssigkeitsleitung beeinflusst wird.

D. h. die Oszillationseinheit führt eine mechanische Schwingung aufgrund der elektrischen Anregung und einer damit verbundenen volumetrischen Ausdehnung in zumindest einer Raumrichtung aus. Die elektromechanische Schwingung der Oszillationseinheit regt aufgrund des Kontakts die Flüssigkeitsleitung und folglich auch den darin befindlichen Inhalt zu einer Schwingung an. Die mechanischen Eigenschaften des zur Schwingung angeregten Systems aus mindestens Oszillationseinheit, Flüssigkeitsleitung und Inhalt der Flüssigkeitsleitung beeinflusst die Impedanz der Oszillationseinheit, sodass die Impedanz der Oszillationseinheit Rückschlüsse auf den Inhalt der Flüssigkeitsleitung ermöglicht.

In anderen Worten beeinflusst das Gasvolumen in dem Abschnitt der Flüssigkeitsleitung ein Schwingverhalten des Systems, welches als Feder-Masse-Dämpfer-System angesehen werden kann. Das Schwingverhalten des Systems beeinflusst die Impedanz der Oszillationseinheit, weshalb das Gasvolumen im Abschnitt der Flüssigkeitsleitung anhand der Impedanz der Oszillationseinheit bestimmbar ist.

Die Impedanz ist eine elektromechanische Impedanz, d. h. eine elektrische Impedanz der elektromechanisch schwingenden Oszillationseinheit. Diese wird auch als Wechselstromwiderstand bezeichnet und lässt sich in ein Amplitudenverhältnis der Wechselspannung und des Wechselstroms und eine Phasenverschiebung zwischen Wechselspannung und Wechselstrom aufspalten.

Der Inhalt der Flüssigkeitsleitung kann eine Flüssigkeit, eine Flüssigkeit mit einer Gasphase oder eine Gasphase sein. Der Anteil des Gasvolumens an der Flüssigkeit im Abschnitt der Flüssigkeitsleitung ist folglich durch die Impedanz klassifizierbar. Der Abschnitt der Flüssigkeitsleitung ergibt sich aus einer Länge eines Kontaktbereichs der Oszillationseinheit mit der Flüssigkeitsleitung.

Die medizinische Vorrichtung kann vorzugsweise eine Vorrichtung zur Flüssigkeitsförderung oder eine Vorrichtung zur Überwachung, Regelung oder Steuerung der Vorrichtung zur Flüssigkeitsförderung sein. Die Oszillationseinheit kann direkt an der Flüssigkeitsleitung anliegen oder von einer Ummantelung, zumindest teilweise, umgeben sein. Die Oszillationseinheit kann auch an einem Gehäuseabschnitt fixiert sein, an welchem die Flüssigkeitsleitung anliegt.

Die Flüssigkeitsleitung ist vorzugsweise ein flexibler Schlauch, insbesondere aus transparentem Polyvinylchlorid oder Polyethylen. Ein Durchmesser der Flüssigkeitsleitung kann zwischen 2 mm und 20 mm, vorzugsweise zwischen 3 mm und 8 mm, insbesondere 4 mm, liegen.

Die Ansteuereinheit erzeugt eine elektrische Spannung, vorzugsweise eine sinusförmige, insbesondere frequenzvariable, Wechselspannung und beaufschlagt damit die Oszillationseinheit. Dazu ist die Ansteuereinheit vorzugsweise elektrisch mit der Oszillationseinheit verbunden, d. h. verdrahtet.

Die Ansteuereinheit und die Auswerteeinheit können vorzugsweise in derselben Einheit bzw. Logikeinheit kombiniert sein.

Vorzugsweise kann die Auswerteeinheit ausgebildet sein, einen durch die Oszillationseinheit fließenden elektrischen Strom zu erfassen.

Die von der Ansteuereinheit angelegte elektrische Spannung führt zu dem elektrischen Strom, der durch die Oszillationseinheit fließt. Der Strom, d. h. die Stromstärke, kann von der Auswerteeinheit oder einer mit dieser verbundenen Messeinheit erfasst werden. Unter Erfassen kann ein Bestimmen eines Messwerts des elektrischen Stroms, ein Aufzeichnen und ein Abspeichern des Messwerts verstanden werden. Dadurch kann die Ansteuereinheit den Messwert des Stroms, welcher durch das Oszillationselement fließt, verarbeiten.

Vorzugsweise ist die Auswerteeinheit ausgebildet, mithilfe einer Fouriertransformation der angelegten elektrischen Spannung und des erfassten elektrischen Stroms die Impedanz der Oszillationseinheit zu berechnen.

Die Fouriertransformation kann vorzugsweise als diskrete Fouriertransformation (DFT) oder schnelle Fouriertransformation (FFT) ausgeführt werden.

Die Auswerteeinheit kann die von der Ansteuereinheit angelegte elektrische Spannung erfassen oder von der Ansteuereinheit eine Information über die angelegte elektrische Spannung erhalten.

Dadurch kann die Impedanz des Oszillationselements der angelegten elektrischen Spannung, d.h. insbesondere einer Frequenz der angelegten Wechselspannung, zugeordnet werden.

Vorzugsweise ist die Ansteuereinheit ausgebildet, die Oszillationseinheit über einen vorgegebenen Frequenzbereich anzuregen. Die Auswerteeinheit kann ausgebildet sein, einen Amplitudengang und/oder einen Phasengang der Impedanz über den vorgegebenen Frequenzbereich zu erfassen. Der vorgegebene Frequenzbereich kann bevorzugt zwischen 1,7 MHz und 2,1 MHz liegen.

D. h. die Ansteuereinheit kann die Oszillationseinheit mit elektrischem Wechselstrom verschiedener Frequenzen innerhalb des vorgegebenen Frequenzbereichs beaufschlagen.

Der Amplitudengang kann das Amplitudenverhältnis, d. h. einen Realteil, der komplexwertigen Impedanz über den vorgegebenen Frequenzbereich darstellen. Der Phasengang kann das Argument, d. h. die Phasenverschiebung, der komplexwertigen Impedanz über den vorgegebenen Frequenzbereich darstellen.

Die Erfassung der Impedanz in Abhängigkeit der Frequenz der Anregung der Oszillationseinheit kann als elektromechanische Impedanzspektroskopie bezeichnet werden.

Das Gasvolumen im Abschnitt der Flüssigkeitsleitung kann bei unterschiedlichen Frequenzen der Anregung einen unterschiedlich großen Einfluss auf die Impedanz der Oszillationseinheit haben. Da die Anregung und die Erfassung bei verschiedenen Frequenzen innerhalb des vorgegebenen Frequenzbereichs erfolgt, können auch die für das zu bestimmende vorliegende Gasvolumen relevanten Frequenzen ausgewertet werden. Dadurch kann das im Abschnitt der Flüssigkeitsleitung vorhandenen Gasvolumen genauer bestimmt werden, als dies bei der Auswertung einer einzelnen Frequenz möglich ist.

Es ist weiterhin vorteilhaft, dass die Auswerteeinheit einen Speicher aufweist. In dem Speicher können Referenzwerte für die Impedanz oder mindestens ein Referenzverlauf für den Amplitudengang und/oder den Phasengang gespeichert sein. Die Auswerteeinheit kann ausgebildet sein, das Gasvolumen durch Vergleichen der bestimmten, d.h. gemessenen, Impedanz(en) mit den Referenzwerten oder des Amplitudengangs und/oder des Phasengangs mit dem jeweiligen Referenzverlauf zu bestimmen.

Die Referenzwerte können Werte der Impedanz für bestimme Frequenzen innerhalb des vorgegebenen Frequenzbereichs sein.

Der Referenzverlauf kann ein Werteverlauf des Amplitudenverhältnisses und/oder der Phasenverschiebung über den vorgegebenen Frequenzbereich sein.

Mit den Referenzwerten und/oder dem Referenzverlauf kann ein Zustand des Inhalts in dem Abschnitt der Flüssigkeitsleitung und/oder ein Wert des in dem Abschnitt der Flüssigkeitsleitung vorhandenen Gasvolumens verknüpft sein. Der Zustand kann auch eine Abwesenheit von Gas, d.h. ein Gasvolumen von 0, sein. Der Wert kann insbesondere eine Volumenangabe sein. Ferner kann der Zustand und/oder der Wert auf dem Speicher gespeichert sein.

Der Vergleich der bestimmten, d.h. gemessenen, Impedanz mit den Referenzwerten oder des Amplitudengangs und/oder des Phasengangs mit dem jeweiligen Referenzverlauf kann anhand eines Korrelationskoeffizienten erfolgen. Der Korrelationskoeffizient stellt ein Maß für den Grad des linearen Zusammenhangs zwischen zwei intervallskalierten Merkmalen dar. Dazu wird für jede betrachtete Frequenz die bestimmte Impedanz, deren Amplitudenverhältnis oder deren Phasenverschiebung in Bezug zu dessen Mittelwert aller betrachteter Frequenzen gesetzt und mit dem Referenzwert zu der Frequenz aus den Referenzwerten oder dem Referenzverlauf, welcher mit dessen Mittelwert aller betrachteter Frequenzen in Bezug gesetzt wird, korreliert. Ein Korrelationskoeffizient von 1 kann ein Übereinstimmen des Zustands des Inhalts in dem Abschnitt der Flüssigkeitsleitung mit dem Zustand, welcher mit den Referenzwerten oder mit dem Referenzverlauf verknüpft ist, und/oder ein Übereinstimmen des Gasvolumens im Abschnitt der Flüssigkeitsleitung mit dem Wert, welcher mit den Referenzwerten oder mit dem Referenzverlauf verknüpft ist, darstellen.

Die Bestimmung des Gasvolumens im Abschnitt der Flüssigkeitsleitung kann anhand des Korrelationskoeffizienten erfolgen. Dazu kann der Korrelationskoeffizient mit den Referenzwerten oder dem Referenzverlauf, welcher mit einem bestimmten Zustand, insbesondere dem Zustand der Abwesenheit von Gas im Abschnitt der Flüssigkeitsleitung und/oder mit dem Wert des Gasvolumens von 0 verknüpft ist, bestimmt werden und anhand dessen das Gasvolumen im Abschnitt der Flüssigkeitsleitung bestimmt werden. Ebenso können Korrelationskoeffizienten mehrerer Referenzwerte oder Referenzverläufe, welche mit verschiedenen Zuständen und/oder Werten verknüpft sind, bestimmt werden und das Gasvolumen anhand des Zustands oder des Werts der Referenzwerte oder des Referenzverlaufs dessen Korrelationskoeffizient am nächsten an 1 ist, bestimmt werden.

Dadurch kann anhand eines Verlaufs der bestimmten Impedanz, d.h. eines Verlaufs des Amplitudengangs und/oder des Phasengangs, über den vorgegebenen Frequenzbereich das Gasvolumen im Abschnitt der Flüssigkeitsleitung bestimmt werden.

Nach einem optionalen Aspekt der Offenbarung ist die Ansteuereinheit ausgebildet die Oszillationseinheit mit einer oder mehreren vorgegeben Frequenzstützstellen anzuregen. Die Frequenzstützstellen können bevorzugt zwischen 1,7 MHz und 2,1 MHz liegen. Die Auswerteeinheit kann ausgebildet sein, die Impedanz der einen oder mehreren vorgegebenen Frequenzstützstellen zu erfassen. Die Auswerteeinheit kann einen Speicher aufweisen, in dem Referenzwerte für die Impedanz gespeichert sind. Die Auswerteeinheit kann ausgebildet sein, das Gasvolumen durch Vergleichen der bestimmten Impedanz oder der bestimmen Impedanzen mit den Referenzwerten zu bestimmen.

Die Frequenzstützstellen können einzelne Frequenzen sein, bei denen die Impedanz der Oszillationseinheit von dem Gasvolumen im Abschnitt der Flüssigkeitsleitung beeinflusst wird. Die Frequenzstützstellen können vorzugsweise Frequenzen sein, an denen eine Änderung des Gasvolumens zu einer deutlichen Änderung der Impedanz führt.

Dadurch kann die Bestimmung des Gasvolumens im Abschnitt der Flüssigkeitsleitung auch anhand von einzelnen Frequenzen der Anregung erfolgen.

Vorzugsweise weist die Auswerteeinheit eine Mustererkennungseinheit auf, die ausgebildet ist, das Gasvolumen durch Eingeben der bestimmten Impedanz oder des Amplitudengangs und/oder des Phasengangs zu bestimmen.

Die Mustererkennungseinheit kann Regelmäßigkeiten, Wiederholungen, Ähnlichkeiten oder Gesetzmäßigkeiten der bestimmten Impedanz oder des bestimmten Amplitudengangs und/oder des bestimmten Phasengangs erkennen.

Dadurch kann die Bestimmung des Gasvolumens im Abschnitt der Flüssigkeitsleitung anhand von Mustern erfolgen.

Die Mustererkennung kann offline bzw. statisch während einer Entwicklungsphase trainiert werden. Ferner kann die Mustererkennungseinheit adaptiv ausgebildet sein und/oder online im Betrieb, bei einem Setwechsel, d. h. einem Wechsel der Flüssigkeitsleitung, oder nach einem Entlüften adaptiert werden.

Die Mustererkennungseinheit kann mit einer Bestimmung des Gasvolumens unter Berücksichtigung von Referenzwerten und/oder dem Referenzverlauf und/oder dem Korrelationskoeffizienten kombiniert werden. Demnach stehen zwei Werte für das Gasvolumen zur Verfügung, die beispielsweise zum Zweck einer höheren Robustheit gemittelt werden können.

Nach einem optionalen Aspekt der Offenbarung weist die Oszillationseinheit mindestens ein Oszillationselement auf. Das Oszillationselement kann insbesondere ein Piezoelement sein.

Das Oszillationselement kann ein elektromechanischer Erreger und/oder Sensor sein. Dazu können Materialien verwendet werden, die bei Anlegen einer elektrischen Spannung eine Längenänderung und/oder Volumenänderung erfahren. Vorzugsweise können dazu Piezoelemente, wie Piezokeramiken oder Piezokristalle, verwendet werden.

Dadurch kann eine Koppelung der elektrischen Anregung und des mechanischen Schwingverhaltens erreicht werden.

Vorzugsweise weist die Oszillationseinheit ein weiteres Oszillationselement auf, das in einem vorgegebenen Winkel versetzt zu dem Oszillationselement mit der Flüssigkeitsleitung in Kontakt bringbar ist. Bevorzugt kann der Winkel zwischen den Oszillationselementen 90° oder 180° betragen.

D. h. die Oszillationselemente können die Flüssigkeitsleitung von zwei Seiten, vorzugsweise von zwei angrenzenden Seiten, d.h. in zwei Raumrichtungen, oder von zwei gegenüberliegenden Seiten kontaktieren. Die Ansteuereinheit kann ausgebildet sein, durch Anlegen von elektrischen Spannungen, insbesondere von Wechselspannungen, die Oszillationselemente zu Schwingungen anzuregen. Die Auswerteeinheit kann ausgebildet sein, zwei Impedanzen der angeregten Oszillationselemente zu bestimmen und das Gasvolumen in dem Abschnitt der Flüssigkeitsleitung unter Berücksichtigung der Impedanzen zu bestimmen.

Das eine Oszillationselement kann als Sender, das weitere Oszillationselement kann als Empfänger wirken. Mehrere Oszillationselemente können ferner eine Messung aus zwei Raumrichtungen ausführen.

Dadurch kann die Bestimmung des Gasvolumens anhand einer bestimmten Transmission zwischen Sender und Empfänger und/oder anhand der Messung aus zwei Raumrichtungen erfolgen. Dadurch kann insbesondere das Gasvolumen von Gasblasen, die nicht einen gesamten Querschnitt der Flüssigkeitsleitung ausfüllen, bestimmt werden. Die bestimmte Transmission kann zur Bestimmung des Gasvolumens mit Referenzwerten oder einem Referenzverlauf der Transmission verglichen werden. Ferner können Übertragungseigenschaften wie mechanische Struktureigenschaften oder eine komplexwertige Übertragungsfunktion in Anhängigkeit der Frequenz zur Bestimmung des Gasvolumens im Abschnitt der Flüssigkeitsleitung bestimmt und ausgewertet werden.

Die Oszillationseinheit kann mehrere Oszillationselemente aufweisen. Die Ansteuereinheit kann so ausgebildet sein, die Oszillationselemente gleichzeitig oder nacheinander anzusteuern.

Nach einem weiteren optionalen Aspekt der Offenbarung weist das eine Oszillationselement oder die mehreren Oszillationselemente jeweils zwei Elektrodenpaare auf.

Das eine Elektrodenpaar des Oszillationselements kann als Sender, das weitere Elektrodenpaar des Oszillationselements kann als Empfänger wirken. Mehrere Elektrodenpaare des Oszillationselements können ferner eine Messung aus zwei Raumrichtungen ausführen. Ferner kann eine Oszillationseinheit mit zwei Elektrodenpaaren des Oszillationselements so ausgebildet sein, eine komplexe Signalmodulation zu erzeugen.

Vorzugsweise weist die Oszillationseinheit einen Reflektor auf, welcher auf einer dem Oszillationselement oder den Oszillationselementen gegenüberliegenden Seite der Flüssigkeitsleitung angeordnet ist.

Der Reflektor kann ausgebildet sein, die Flüssigkeitsleitung zu kontaktieren. Der Reflektor kann aus einem Material gefertigt sein, welches eine höhere Dichte als das Material der Ummantelung oder des Gehäuses, in dem die Oszillationseinheit zumindest teilweise vorhanden ist, aufweist. Dadurch kann die elektromechanische Schwingung der Oszillationseinheit, welche von der Flüssigkeitsleitung an den Reflektor übertragen werden kann, von diesem reflektiert werden. Die Bestimmung des Gasvolumens im Abschnitt der Flüssigkeitsleitung kann auch anhand einer Reflexion und/oder eines Reflektionsgrades der angeregten Schwingung erfolgen. Die Reflexion und/oder der Reflexionsgrad kann mit Referenzwerten verglichen werden oder mit einem Referenzverlauf der Reflexion und/oder des Reflexionsgrads korreliert werden.

Es ist weiterhin vorteilhaft, dass die medizinische Vorrichtung eine Aufnahme für die Flüssigkeitsleitung und eine Klappe zum Sichern der Flüssigkeitsleitung in der Aufnahme aufweist. Die Oszillationseinheit kann in der Aufnahme und/oder in der Klappe angeordnet sein.

Die Aufnahme kann ausgebildet sein, die Flüssigkeitsleitung formschlüssig und/oder kraftflüssig in einer Position zu klemmen, an der die Oszillationseinheit die Flüssigkeitsleitung kontaktiert.

Die Klappe kann ausgebildet sein, in einer geöffneten Position eine Entnahme der Flüssigkeitsleitung aus der Aufnahme zu ermöglichen. Die Klappe kann ausgebildet sein, in einer geschlossenen Position eine Entnahme der Flüssigkeitsleitung aus der Aufnahme zu verhindern.

Dadurch kann ein versehentliches Entfernen der Flüssigkeitsleitung aus der medizinischen Vorrichtung, insbesondere aufgrund einer Bewegung der Flüssigkeitsleitung oder der medizinischen Vorrichtung, verhindert werden.

Die Bestimmung des Gasvolumens in dem Abschnitt der Flüssigkeitsleitung kann bei geschlossener Klappe erfolgen.

Vorzugsweise ist die medizinische Vorrichtung eine medizinische Pumpe oder eine Klemme. Die medizinische Pumpe kann insbesondere eine Infusionspumpe sein.

Die medizinische Pumpe kann einer Förderung von Flüssigkeiten dienen. Die Klemme kann so ausgebildet sein, dass sie an der Flüssigkeitsleitung anbringbar ist.

Ferner kann die Auswerteeinheit ausgebildet sein, dass diese beim Bestimmen eines vorgegebenen Gasvolumens oder eines vorgegebenen geförderten Gesamtgasvolumens ein Signal ausgibt. Das vorgegebene Gasvolumen kann beispielsweise auf 10 µl festgelegt sein. Das Signal kann zum Stoppen der Förderung der Flüssigkeit herangezogen werden. Das Signal kann auch zum Ausgeben einer akustischen und/oder optischen Warnung über eine entsprechende Ausgabeeinrichtung herangezogen werden.

Dadurch kann die Förderung der Flüssigkeit unterbunden werden, wenn bestimmt wird, dass sich im Abschnitt der Flüssigkeitsleitung eine Gasblase eines vorgegeben Volumens oder größer befindet. Ferner kann die Förderung unterbunden werden, wenn ein Gesamtvolumen eines Gases, welches durch die Flüssigkeitsleitung gefördert wurde, in einer vorgegebenen Zeitspanne oder seit Beginn der Förderung einen vorgegebenen Wert überschreitet. Das Gesamtgasvolumen kann insbesondere aus den bestimmten Gasvolumina im Abschnitt der Flüssigkeitsleitung, dem Volumen der Flüssigkeitsleitung im Abschnitt der Flüssigkeitsleitung und einer Fördergeschwindigkeit integriert werden. Die Integration kann als Summe von Gasvolumina pro Zeiteinheit approximiert werden.

Die Bestimmung des Gasvolumens im Abschnitt der Flüssigkeitsleitung kann wiederkehrend, insbesondere vor und während der Förderung, erfolgen.

Neben dem Korrelationskoeffizienten kann die Bestimmung des Gasvolumens im Abschnitt der Flüssigkeitsleitung durch Auswertung der bestimmten Impedanz über eine Integration, eine Normierung oder eine relative Änderung der Impedanz, einer Matrix von Referenzwerten oder einem Schwellwert erfolgen.

Ferner kann ein Verfahren zur Erfassung von Gasvolumen in einem Abschnitt einer Flüssigkeitsleitung folgende Schritten umfassen. Anlegen einer elektrischen Spannung, insbesondere einer, vorzugsweise frequenzvariablen, Wechselspannung, an eine Oszillationseinheit, Messen der elektrischen Spannung und eines elektrischen Stromes, welcher durch die Oszillationseinheit fließt. Bestimmen einer Impedanz der Oszillationseinheit mithilfe einer Fouriertransformation der gemessenen elektrischen Spannung und des gemessenen elektrischen Stroms. Vergleichen oder Korrelieren der Impedanz mit einem Referenzwert. Ausgeben eines Ausgabewerts.

Neben der Bestimmung des Gasvolumens im Abschnitt der Flüssigkeitsleitung können Betriebszustände der medizinischen Vorrichtung, wie eine Abwesenheit der Flüssigkeitsleitung, eine Füllung der Flüssigkeitsleitung mit Wasser, eine Anwesenheit von Gasblasen in der Flüssigkeitsleitung, eine Öffnung oder Schließung der Klappe, ein Auftreten eines Defekts an dem Oszillationselement, eine Empfehlung eines Tausches des Oszillationselements, Mikrorisse, Alterung oder Verschmutzung, bestimmt werden. Dadurch können Entscheidungswerte für weitere Steuerungslogiken bereitgestellt werden. Ferner kann eine vorsorgliche Wartung angefragt werden.

Die Referenzwerte oder der Referenzverlauf kann bei einem bekannten Zustand im Abschnitt der Flüssigkeitsleitung bestimmt werden. Die Referenzwerte oder der Referenzverlauf kann der medizinischen Vorrichtung vorgegeben werden oder manuell an dieser trainiert werden. Dies kann bei einer Herstellung und/oder bei einer Funktionsüberprüfung und/oder bei einer Wartung erfolgen. Die Referenzwerte oder der Referenzverlauf kann ferner adaptiv angepasst werden oder über eine Kommunikation von außen vorgegeben werden.

Verschiedene Flüssigkeiten und/oder verschiedene Flüssigkeitsleitungen können verschiedene Referenzwerte oder Referenzverläufe zur Bestimmung des Gasvolumens in der Flüssigkeitsleitung erfordern. Dazu kann eine Eingabe oder Vorgabe einer Art der Flüssigkeit und/oder einer Art der Flüssigkeitsleitung in die medizinische Vorrichtung notwendig sein. Die Eingabe kann manuell über ein Bedienfeld erfolgen. Die medizinische Vorrichtung kann ferner ausgebildet sein, die Art der Flüssigkeit und/oder der Flüssigkeitsleitung auch selbständig bzw. automatisch zu erkennen.

### Kurzbeschreibung der Figuren

Fig. 1 zeigt eine schematische Darstellung der medizinischen Vorrichtung gemäß eines ersten Ausführungsbeispiels der vorliegenden Offenbarung;
Fig. 2 zeigt eine schematische Darstellung der medizinischen Vorrichtung mit Darstellung eines Oszillationselements;
Fig. 3 zeigt eine schematische Darstellung der medizinischen Vorrichtung mit einem Oszillationselement mit zwei Elektrodenpaaren;
Fig. 4 zeigt eine schematische Darstellung der medizinischen Vorrichtung mit einem Reflektor;
Fig. 5 zeigt eine medizinische Vorrichtung mit zwei Oszillationselementen;
Fig. 6 zeigt eine schematische Darstellung einer weiteren Ausführungsform der medizinischen Vorrichtung in Form einer Klemme in einer Ansicht mit horizontaler Längsachse einer Flüssigkeitsleitung;
Fig. 7 zeigt eine schematische Darstellung der weiteren Ausführungsform der medizinischen Vorrichtung in Form der Klemme in einer Ansicht mit Längsachse der Flüssigkeitsleitung orthogonal zur Bildebene;
Fig. 8 zeigt eine isometrische Ansicht einer weiteren Ausführungsform der medizinischen Vorrichtung in Form einer Infusionspumpe mit einer Gasblase mit 8 µl Volumen;
Fig. 9 zeigt die isometrische Ansicht der weiteren Ausführungsform der medizinischen Vorrichtung in Form der Infusionspumpe mit einer Gasblase mit 24 µl Volumen;
Fig. 10 zeigt einen Amplitudengang und einen Phasengang der elektromechanischen Impedanz der Oszillationseinheit der medizinischen Vorrichtung; und
Fig. 11 zeigt eine Tabelle mit Korrelationskoeffizienten.

### Detaillierte Beschreibung von Ausführungsformen

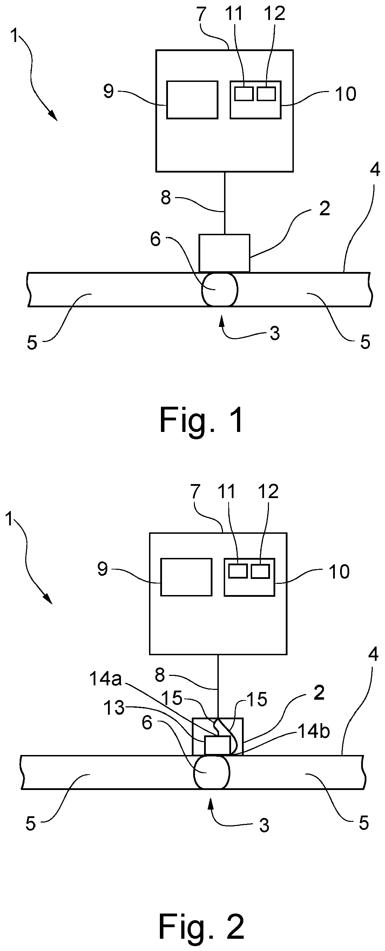

Fig. 1 zeigt eine offenbarungsgemäße medizinische Vorrichtung 1. Die medizinische Vorrichtung 1 weist eine Oszillationseinheit 2 auf, welches einen Abschnitt 3 einer Flüssigkeitsleitung 4 von einer Seite kontaktiert. In der Flüssigkeitsleitung 4 befinden sich eine Flüssigkeit 5 und eine Gasblase 6. Die Gasblase 6 befindet sich im Abschnitt 3 der Flüssigkeitsleitung 4. Die Oszillationseinheit 2 ist mit einer Logikeinheit 7 über eine Transferleitung 8 verbunden. Die Logikeinheit 7 weist eine Ansteuereinheit 9 und eine Auswerteeinheit 10 auf. Die Ansteuereinheit 9 und die Auswerteeinheit 10 kommunizieren mit der Oszillationseinheit 2 über die Transferleitung 8. Die Auswerteeinheit 10 umfasst einen Speicher 11 und eine Mustererkennungseinheit 12.

Fig. 2 zeigt eine Ausführungsform der offenbarungsgemäßen medizinischen Vorrichtung 1 aus Fig. 1 mit einem in der Oszillationselement 13 vorhandenen Oszillationselement 13. Das Oszillationselement 13 ist innerhalb der Oszillationseinheit 2 ausgebildet. Am Oszillationselement 13 sind zwei Elektroden 14a, 14b eins Elektrodenpaars 14 ausgebildet, welche über elektrische Leitungen 15 mit der Transferleitung 8 verbunden sind.

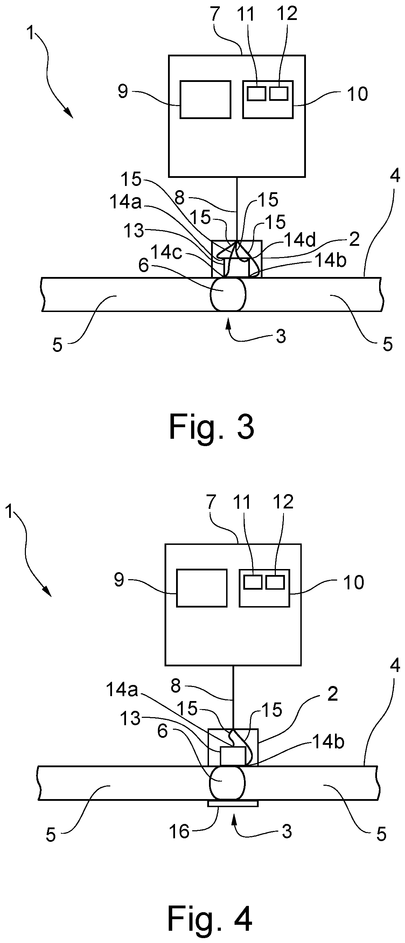

Fig. 3 zeigt eine Ausführungsform der offenbarungsgemäßen medizinischen Vorrichtung 1 aus Fig. 1 mit dem innerhalb der Oszillationseinheit 2 ausgebildeten Oszillationselement 13. Am Oszillationselement 13 sind zwei Elektrodenpaare 14 ausgebildet. Jedes Elektrodenpaar 14 weist zwei Elektroden 14a, 14b, 14c, 14d auf, die jeweils an einer gegenüberliegenden Seite ausgebildet sind. Die beiden Elektroden 14c, 14d des zweiten Elektrodenpaars 14 sind jeweils an gegenüberliegenden Seiten ausgebildet, welche an die Seiten angrenzen, an welchen die Elektroden 14a, 14b des ersten Elektrodenpaars 14 ausgebildet sind.

Fig. 4 zeigt eine Ausführungsform der offenbarungsgemäßen medizinischen Vorrichtung 1 aus Fig. 2, bei der die Oszillationseinheit 2 einen Reflektor 16 aufweist. Der Reflektor 16 ist auf einer dem Oszillationselement 13 gegenüberliegenden Seite der Flüssigkeitsleitung 4 angeordnet und liegt an dem Abschnitt 3 der Flüssigkeitsleitung 4 an.

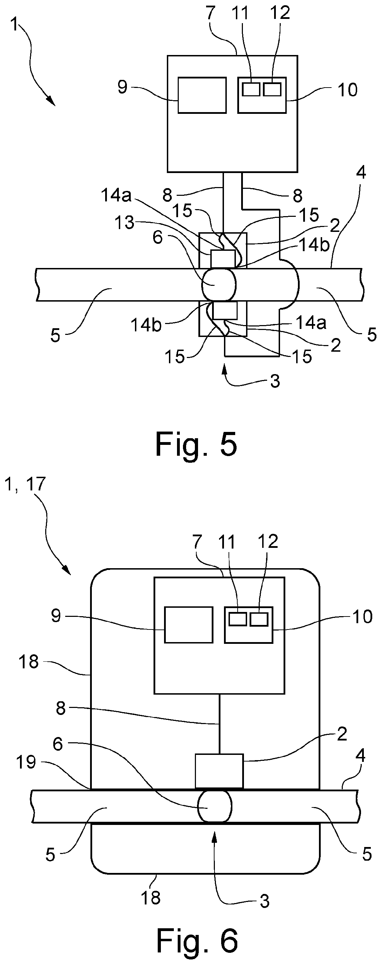

Fig. 5 zeigt eine Ausführungsform der offenbarungsgemäßen medizinischen Vorrichtung 1 aus Fig. 2, bei der die Oszillationseinheit 2 zwei Oszillationselemente 13 aufweist. Das zweite Oszillationselement 13 ist auf einer dem ersten Oszillationselement 13 gegenüberliegenden Seite der Flüssigkeitsleitung 4 ausgebildet und liegt an dem Abschnitt 3 der Flüssigkeitsleitung 4 an. Die beiden Oszillationselemente 13 sind innerhalb der Oszillationseinheit 2 angeordnet. Diese ist dazu C-förmig ausgebildet und hintergreift die Flüssigkeitsleitung 4. Das zweite Oszillationselement 13 ist mit einer separaten elektrischen Leitung 15 mit einer separaten Transferleitung 8 verbunden. Die Transferleitungen 8 verbinden die Oszillationseinheit 2 mit der Logikeinheit 7.

Fig. 6 zeigt eine weitere Ausführungsform der offenbarungsgemäßen medizinischen Vorrichtung 1 aus Fig. 1 in Form einer Klemme 17. In der Ansicht der Fig. 6 liegt eine Langsachse der Flüssigkeitsleitung 4 horizontal in der Bildebene. Die Logikeinheit 7 mit Ansteuereinheit 9 und Auswerteeinheit 10 mit Speicher 11 und Mustererkennungseinheit 12 sowie die Transferleitung 8 und die Oszillationseinheit 2 werden von einem Klemmengehäuse 18 umschlossen. Zur Aufnahme der Flüssigkeitsleitung 4 weist das Klemmengehäuse 18 eine Aufnahme 19 in Form einer Ausnehmung auf. Die Flüssigkeitsleitung 4 liegt entlang der Aufnahme 19 an dem Klemmengehäuse 18 an. Die Aufnahme 19 ist vorgesehen und ausgebildet, die Flüssigkeitsleitung 4 formschlüssig zu klemmen. Die Oszillationseinheit 2 liegt auf einer der Flüssigkeitsleitung 4 gegenüberliegenden Seite an dem Klemmengehäuse 18 an.

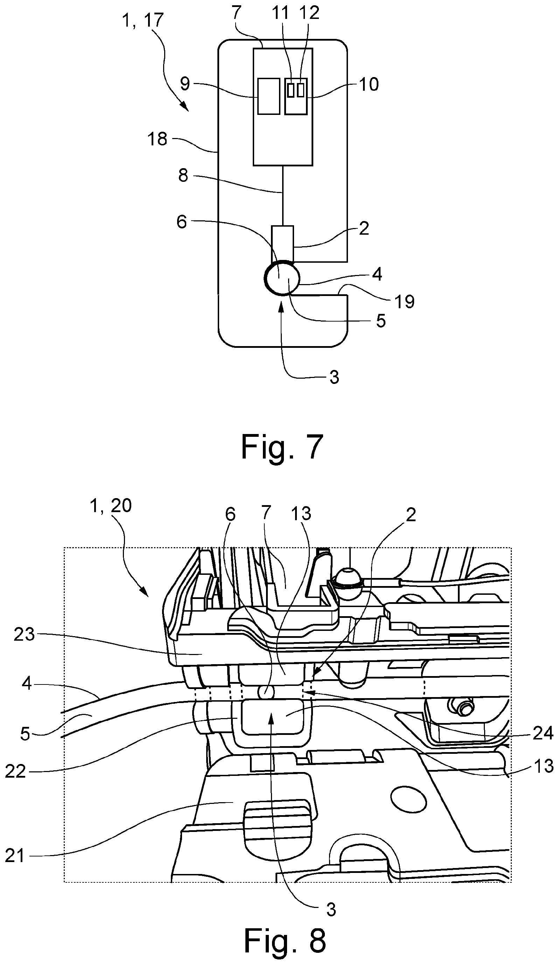

Fig. 7 zeigt die Ausführungsform der offenbarungsgemäßen medizinischen Vorrichtung 1 aus Fig. 1 in Form der Klemme 17. In der Ansicht der Fig. 7 liegt die Langsachse der Flüssigkeitsleitung 4 orthogonal zur Bildebene. Die Aufnahme 19 weist in dieser Ansicht einen C-förmigen Querschnitt auf. Die Flüssigkeitsleitung 4 liegt an einem Scheitelpunkt der Aufnahme 19 an dem Klemmengehäuse 18 an. Die Aufnahme 19 ist vorgesehen und ausgebildet, die Flüssigkeitsleitung 4 an dem Scheitelpunkt formschlüssig zu klemmen. Die Oszillationseinheit 2 liegt auf einer der Flüssigkeitsleitung 4 gegenüberliegenden Seite an dem Klemmengehäuse 18 an.

Fig. 8 zeigt eine weitere Ausführungsform der medizinischen Vorrichtung 1 in Form einer Infusionspumpe 20. Die Infusionspumpe 20 weist eine Oszillationseinheit 2 mit zwei Oszillationselementen 13 auf, welche von einem Oszillationsgehäuse 22 teilweise ummantelt sind. Zwischen den Oszillationselementen 13 befindet sich die Flüssigkeitsleitung 4, sodass die Oszillationselemente 13 die Flüssigkeitsleitung 4 auf zwei gegenüberliegenden Seiten kontaktieren. Das Oszillationsgehäuse 22 bildet eine Aufnahme 24 aus, welche die Flüssigkeitsleitung 4 formschlüssig klemmt. Im Abschnitt 3 der Flüssigkeitsleitung 4 befindet sich eine Gasblase 6 mit einem Volumen von 8 µl innerhalb der Flüssigkeitsleitung 4. Die restliche Flüssigkeitsleitung 4 ist vollständig mit der Flüssigkeit 5 gefüllt. Innerhalb eines Infusionspumpengehäuses 23 der Infusionspumpe 20 ist die Logikeinheit 7 mit der in Fig. 8 nicht dargestellten Ansteuereinheit 9 und der in Fig. 8 nicht dargestellten Auswerteeinheit 10 mit Speicher 11 und Mustererkennungseinheit 12 integriert. Diese sind mithilfe der in Fig. 8 nicht dargestellten Transferleitung 8 mit der Oszillationseinheit 2 verbunden. An dem Infusionspumpengehäuse 23 ist eine Klappe 21 schwenkbar ausgebildet. Im geöffneten Zustand der Klappe 21 kann die Flüssigkeitsleitung 4 aus der Infusionspumpe 20 entnommen werden. Bei geschlossener Klappe 21 verhindert diese eine Entnahme der Flüssigkeitsleitung 4 aus der Aufnahme 24 bzw. der Infusionspumpe 20. Kanten des Oszillationsgehäuses 22 und des Infusionspumpengehäuses 23, welche hinter der transparenten Flüssigkeitsleitung 4 liegen, sind in Fig. 8 gestrichelt dargestellt.

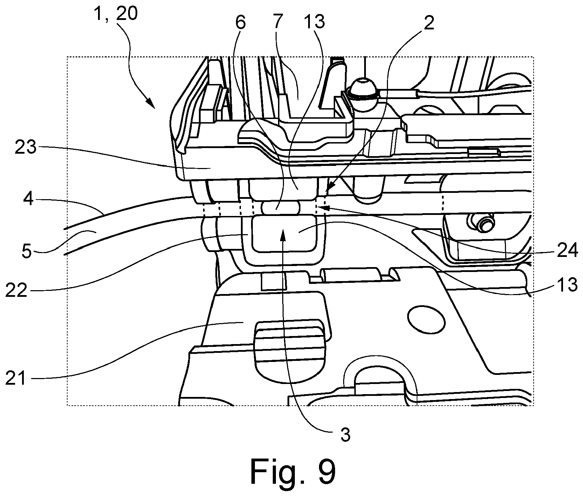

Fig. 9 zeigt die weitere Ausführungsform der medizinischen Vorrichtung 1 in Form der Infusionspumpe 20 aus Fig. 8. In dieser Darstellung ist eine Gasblase 6 mit einem Volumen von 24µl innerhalb der Flüssigkeitsleitung 4 vorhanden.

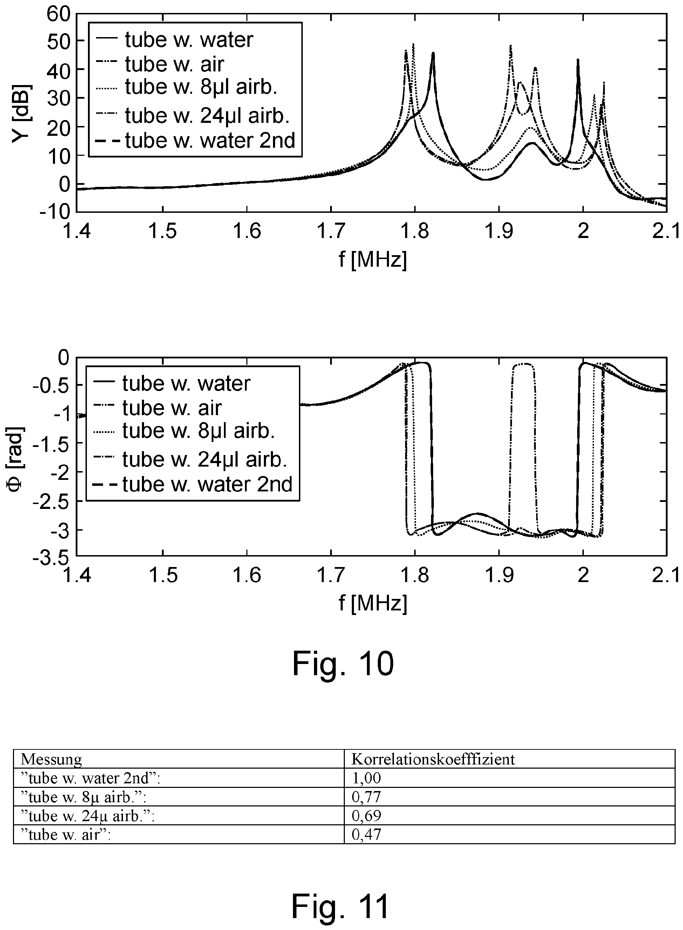

Fig. 10 zeigt ein Bode-Diagramm, d.h. ein Diagramm eines Amplitudengangs und ein Diagramm eines Phasengangs, der Impedanz der Oszillationseinheit 2. Die beiden Diagramme tragen ein Amplitudenverhältnis in Dezibel (dB) bzw. eine Phasenverschiebung in Radiant (rad) über eine Frequenz zwischen 1,4 Megahertz (MHz) und 2,1 MHz an. Beide Diagramme zeigen jeweils fünf Verläufe des Amplitudenverhältnisses und der dazugehörigen Phasenverschiebung. Jedem der Verläufe ist ein Zustand zugeordnet. Vier Verläufe entsprechen verschiedenen Zuständen im Abschnitt 3 der Flüssigkeitsleitung 4. Der fünfte Verlauf repräsentiert eine Vergleichsmessung des ersten Verlaufs. Ein erster Verlauf ist mit einer durchgezogenen Linie (-) dargestellt und zeigt die Impedanz der Oszillationseinheit 2, wenn der Abschnitt 3 der Flüssigkeitsleitung 4 mit Wasser gefüllt ist. Ein zweiter Verlauf ist mit einer Strich-Punkt-Punkt-Strich-Linie (- -) dargestellt und zeigt die Impedanz der Oszillationseinheit 2, wenn der Abschnitt 3 der Flüssigkeitsleitung 4 mit Luft gefüllt ist. Ein dritter Verlauf ist mit einer gepunkteten Linie (····) dargestellt und zeigt die Impedanz der Oszillationseinheit 2, wenn der Abschnitt 3 der Flüssigkeitsleitung 4 mit einer Luftblase mit einem Volumen von 8 µl in Wasser gefüllt ist. Ein vierter Verlauf ist mit einer Strich-Punkt-Strich-Linie (- -) dargestellt und zeigt die Impedanz der Oszillationseinheit 2, wenn der Abschnitt 3 der Flüssigkeitsleitung 4 mit einer Luftblase mit einem Volumen von 24 µl in Wasser gefüllt ist. Ein fünfter Verlauf ist mit einer gestrichelten Linie (- -) dargestellt und zeigt erneut die Impedanz der Oszillationseinheit 2, wenn der Abschnitt 3 der Flüssigkeitsleitung 4 mit Wasser gefüllt ist. Alle fünf Verläufe des Amplitudenverhältnisses weisen einen W-förmigen Graph auf. Bei Frequenzen unterhalb von 1,7 MHz und oberhalb von 2,05 MHz tangieren die Verläufe gegen Werte unter 0 dB. Dazwischen weisen die Verläufe drei oder vier lokale Maxima auf. Alle fünf Verläufe der Phasenverschiebung weisen einen W-förmigen Graph auf. Bei Frequenzen unterhalb von 1,7 MHz und oberhalb von 2,05 MHz tangieren die Verläufe gegen Werte von etwa -1 rad. Dazwischen weisen die Verläufe drei oder vier lokale Minima auf. Im Bereich von 1,7 MHz bis 2,05 MHz variieren die ersten vier Verläufe und zeigen eine Abhängigkeit des Resonanzverhaltens vom Gasvolumen im Abschnitt der Flüssigkeitsleitung 4. Der erste und der fünfte Verlauf, d. h. die Verläufe der Zustände mit Wasser, sind deckungsgleich.

Fig. 11 zeigt eine Tabelle mit Korrelationskoeffizienten des Phasengangs für die verschiedenen Zustände im Abschnitt 3 der Flüssigkeitsleitung 4 aus Fig. 10. Als Referenzverlauf für den Korrelationskoeffizienten dient der in Fig. 10 mit der durchgezogenen Linie (-) dargestellte erste Verlauf, bei welchem der Abschnitt 3 der Flüssigkeitsleitung 4 mit Wasser gefüllt ist. Eine zweite Zeile weist dem Verlauf, wenn der Abschnitt 3 der Flüssigkeitsleitung 4 mit Wasser gefüllt ist, einen Korrelationskoeffizienten von 1,00 zu. Eine dritte Zeile weist dem Verlauf, wenn der Abschnitt 3 der Flüssigkeitsleitung 4 mit der Luftblase mit einem Volumen von 8µl in Wasser gefüllt ist, einen Korrelationskoeffizienten von 0,77 zu. Eine vierte Zeile weist dem Verlauf, wenn der Abschnitt 3 der Flüssigkeitsleitung 4 mit der Luftblase mit einem Volumen von 24µl in Wasser gefüllt ist, einen Korrelationskoeffizienten von 0,69 zu. Eine fünfte Zeile weist dem Verlauf, wenn der Abschnitt 3 der Flüssigkeitsleitung 4 mit Luft gefüllt ist, einen Korrelationskoeffizienten von 0,47 zu. Anhand des Korrelationskoeffizienten gegen den Referenzverlauf mit Wasser lässt sich somit das Gasvolumen der Gasblase 6 im Abschnitt 3 der Flüssigkeitsleitung 4 bestimmen.

### Bezugszeichenliste

- 1: medizinische Vorrichtung
- 2: Oszillationseinheit
- 3: Abschnitt
- 4: Flüssigkeitsleitung
- 5: Flüssigkeit
- 6: Gasblase
- 7: Logikeinheit
- 8: Transferleitung
- 9: Ansteuereinheit
- 10: Auswerteeinheit
- 11: Speicher
- 12: Mustererkennungseinheit
- 13: Oszillationselement
- 14: Elektrodenpaar
- 14a, 14b, 14c, 14d: Elektrode
- 15: Elektrische Leitung
- 16: Reflektor
- 17: Klemme
- 18: Klemmengehäuse
- 19: Aufnahme
- 20: Infusionspumpe
- 21: Klappe
- 22: Oszillationsgehäuse
- 23: Infusionspumpengehäuse
- 24: Aufnahme

## Patentansprüche

1. Medizinische Vorrichtung (1), die aufweist:
eine Oszillationseinheit (2), die mit einem Abschnitt (3) einer Flüssigkeitsleitung (4) mittelbar oder unmittelbar in Kontakt bringbar ist,
eine Ansteuereinheit (9), die ausgebildet ist, durch Anlegen einer elektrischen Spannung, insbesondere einer Wechselspannung, die Oszillationseinheit (2) zu einer Schwingung anzuregen, und
eine Auswerteinheit (10), die ausgebildet ist, eine Impedanz der angeregten Oszillationseinheit (2) zu bestimmen und ein Gasvolumen (6) in dem Abschnitt (3) der Flüssigkeitsleitung (4) unter Berücksichtigung der Impedanz zu bestimmen.

2. Medizinische Vorrichtung (1) nach Anspruch 1, **dadurch gekennzeichnet, dass** die Auswerteeinheit (10) ausgebildet ist, einen durch die Oszillationseinheit (2) fließenden elektrischen Strom zu erfassen.

3. Medizinische Vorrichtung (1) nach Anspruch 2, **dadurch gekennzeichnet, dass** die Auswerteeinheit (10) ausgebildet ist, mithilfe einer Fouriertransformation der angelegten elektrischen Spannung und des erfassten elektrischen Stroms die Impedanz der Oszillationseinheit (2) zu berechnen.

4. Medizinische Vorrichtung (1) nach einem der Ansprüche 1 bis 3, **dadurch gekennzeichnet, dass** die Ansteuereinheit (9) ausgebildet ist, die Oszillationseinheit (2) über einen vorgegeben Frequenzbereich, bevorzugt 1,7 MHz bis 2,1 MHz, anzuregen, und
die Auswerteeinheit (10) ausgebildet ist, einen Amplitudengang und/oder einen Phasengang der Impedanz über den vorgegebenen Frequenzbereich zu erfassen.

5. Medizinische Vorrichtung (1) nach Anspruch 4, **dadurch gekennzeichnet, dass** die Auswerteeinheit (10) einen Speicher (11) aufweist, in dem Referenzwerte für die Impedanz oder mindestens ein Referenzverlauf für den Amplitudengang und/oder den Phasengang gespeichert sind, und die Auswerteeinheit (10) ausgebildet ist, das Gasvolumen (6) durch Vergleichen der bestimmten Impedanz mit den Referenzwerten oder des Amplitudengangs und/oder des Phasengangs mit dem jeweiligen Referenzverlauf zu bestimmen.

6. Medizinische Vorrichtung (1) nach einem der Ansprüche 1 bis 3, **dadurch gekennzeichnet, dass** die Ansteuereinheit (9) ausgebildet ist, die Oszillationseinheit (2) mit einer oder mehreren vorgegebenen Frequenzstützstellen, bevorzugt zwischen 1,7 MHz und 2,1 MHz, anzuregen, die Auswerteeinheit (10) ausgebildet ist, die Impedanz der einen oder mehreren vorgegebenen Frequenzstützstellen zu erfassen, und
die Auswerteeinheit (10) einen Speicher (11) aufweist, in dem Referenzwerte für die Impedanz gespeichert sind, und ausgebildet ist, das Gasvolumen (6) durch Vergleichen der bestimmten Impedanz(en) mit den Referenzwerten zu bestimmen.

7. Medizinische Vorrichtung (1) nach einem der Ansprüche 1 bis 6, **dadurch gekennzeichnet, dass** die Auswerteeinheit (10) eine Mustererkennungseinheit (12) aufweist, die ausgebildet ist, das Gasvolumen (6) durch Eingeben der bestimmten Impedanz oder des Amplitudengangs und/oder des Phasengangs zu bestimmen.

8. Medizinische Vorrichtung (1) nach einem der Ansprüche 1 bis 7, **dadurch gekennzeichnet, dass** die Oszillationseinheit (2) mindestens ein Oszillationselement (13), insbesondere ein Piezoelement, umfasst.

9. Medizinische Vorrichtung (1) nach Anspruch 8, **dadurch gekennzeichnet, dass** die Oszillationseinheit (2) ein weiteres Oszillationselement (13), das in einem vorgegebenen Winkel, bevorzugt 90° oder 180°, versetzt zu dem Oszillationselement (13) mit der Flüssigkeitsleitung (4) in Kontakt bringbar ist, aufweist.

10. Medizinische Vorrichtung (1) nach einem der Ansprüche 8 oder 9, **dadurch gekennzeichnet, dass** das/die Oszillationselement(e) (13) zwei Elektrodenpaare (14) aufweist.

11. Medizinische Vorrichtung (1) nach einem der Ansprüche 8 oder 9, **dadurch gekennzeichnet, dass** die Oszillationseinheit (2) einen Reflektor (16) aufweist, welcher auf einer dem/der Oszillationselement(e) (13) gegenüberliegenden Seite der Flüssigkeitsleitung (4) angeordnet ist.

12. Medizinische Vorrichtung (1) nach einem der Ansprüche 1 bis 11, **gekennzeichnet durch** eine Aufnahme (24) für die Flüssigkeitsleitung (4) und eine Klappe (21) zum Sichern der Flüssigkeitsleitung (4) in der Aufnahme (24), wobei die Oszillationseinheit (2) in der Aufnahme (24) und/oder in der Klappe (21) angeordnet ist.

13. Medizinische Vorrichtung (1) nach einem der Ansprüche 1 bis 12, **dadurch gekennzeichnet, dass** das diese eine medizinische Pumpe, insbesondere eine Infusionspumpe (20), oder eine Klemme (17) ist.
